# EUROPEAN PATENT APPLICATION

(11) **EP 3 260 860 A1**
(43) Date of publication of application: **27.12.2017**
(21) Application number: 16175843.8
(22) Date of filing: 23.06.2016
(51) Int. Cl.: G01N 33/00, A61B 5/08, G01N 33/497

(54) **APPARATUS COMPRISING A GRAPHENE BASED SENSOR AND METHOD OF USING THE APPARATUS**

(71) Applicant: Nokia Technologies Oy, 02610 Espoo (FI)
(72) Inventor: LANTZ, Vuokko, 01610 Vantaa (FI); POHJONEN, Helena, 02360 Espoo (FI)
(74) Representative: Nokia EPO representatives

(57) **Abstract**

An apparatus and method where the apparatus comprises: a power source (3) comprising graphene wherein the power source is arranged to be activated by water from a sample (7); at least one sensor (5) comprising graphene arranged to detect one or more analytes (9) within the sample; and wherein the power source is arranged to provide power to the sensor in response to exposure to the sample.

## Description

### TECHNOLOGICAL FIELD

Examples of the disclosure relate to an apparatus and method where the apparatus comprises a graphene based sensor. In particular, they relate to an apparatus and method where the apparatus comprises a graphene based sensor where the sensor is arranged to detect one or more analytes.

### BACKGROUND

Apparatus for sensing analytes are known. The analyte could be the presence of a chemical or group of chemicals within a sample. Such apparatus may comprise sensors which produce a measurable change in electrical properties in response to exposure to the analytes. This enables the apparatus to produce an electrical output indicative of the analyte. Such apparatus could be used in devices such as breathalysers which are arranged to analyse the analytes in a user's breath.

### BRIEF SUMMARY

According to various, but not necessarily all, examples of the disclosure there is provided an apparatus comprising: a power source comprising graphene wherein the power source is arranged to be activated by water from a sample; at least one sensor comprising graphene arranged to detect one or more analytes within the sample; and wherein the power source is arranged to provide power to the sensor in response to exposure to the sample.

The sample may comprise a user's breath.

The apparatus may comprise at least one filter arranged to remove analytes from the sample before the sensor is exposed to the sample.

The apparatus may comprise at least one filter arranged to convert a first analyte into a second analyte before the sensor is exposed to the sample.

The apparatus may comprise an ultraviolet light source arranged to expose the at least one sensor to ultraviolet light. The power source may be arranged to provide power to the ultraviolet light source.

The apparatus may comprise a plurality of sensors. The plurality of sensors may be arranged to have different sensitivities. The plurality of sensors may be arranged to detect different analytes.

The apparatus may comprise one or more indicators arranged to show an output of the sensor. The power source may be arranged to provide power to the one or more indicators.

The apparatus may comprise a chamber arranged to house the power source and the one or more sensors. The chamber may comprise a channel arranged to enable the sample to be introduced into the chamber.

The power source may comprise a first electrode comprising graphene oxide which is arranged to generate protons in response to water from the sample.

The at least one sensor may comprise a graphene channel comprising at least one of; graphene, reduced graphene oxide, functionalized graphene, carbon nanotubes.

The sensor may be arranged so that the analyte causes a change in the electrical conductivity of the graphene channel.

According to various, but not necessarily all, examples of the disclosure there is provided a sensing device comprising an apparatus as claimed in any preceding claim. The sensing device may be disposable.

According to various, but not necessarily all, examples of the disclosure there is provided a method comprising: activating a power source comprising graphene wherein the power source is activated by water from a sample; providing power from the power source to at least one sensor comprising graphene in response to exposure to the sample; and using the at least one sensor to detect one or more analytes within the sample.

The sample may comprise a user's breath.

The method may comprise using at least one filter to remove analytes from the sample before the sensor is exposed to the sample.

The method may comprise using at least one filter arranged to convert a first analyte into a second analyte before the sensor is exposed to the sample.

The method may comprise using an ultraviolet light source to expose the at least one sensor to ultraviolet light. The power source may be arranged to provide power to the ultraviolet light source.

The method may comprise a plurality of sensors used to detect the one or more analytes within the sample. The plurality of sensors may be arranged to have different sensitivities. The plurality of sensors may be arranged to detect different analytes.

The method may comprise using one or more indicators to show an output of the sensor. The power source may be arranged to provide power to the one or more indicators.

The power source and the one or more sensors may be housed within a chamber. The method may comprise introducing the sample to the chamber via a channel.

The power source may comprise a first electrode comprising graphene oxide which is arranged to generate protons in response to water from the sample.

The at least one sensor may comprise a graphene channel comprising at least one of; graphene, reduced graphene oxide, functionalized graphene, carbon nanotubes.

The sensor may be arranged so that the analyte causes a change in the electrical conductivity of the graphene channel.

According to various, but not necessarily all, examples of the disclosure there is provided examples as claimed in the appended claims.

### BRIEF DESCRIPTION

For a better understanding of various examples that are useful for understanding the detailed description, reference will now be made by way of example only to the accompanying drawings in which:
Fig. 1 illustrates an apparatus;
Fig. 2 illustrates a power source;
Fig. 3 illustrates a sensor;
Fig. 4 illustrates a sensing device;
Fig. 5 illustrates a sensing device;
Fig. 6 illustrates a filter;
Figs. 7A to 7C illustrate a sensing device in use;
Fig. 8 illustrates a method; and
Fig. 9 illustrates another method.

### DETAILED DESCRIPTION

The Figures illustrate an apparatus 1 comprising: a power source 3 comprising graphene wherein the power source 3 is arranged to be activated by water from a sample 7; at least one sensor 5 comprising graphene arranged to detect one or more analytes 9 within the sample 7; and wherein the power source 3 is arranged to provide power to the sensor 5 in response to exposure to the sample 7.

The apparatus 1 may be for sensing. The apparatus 1 may be for sensing one or more analytes 9 within a sample 7. In some examples the sample 7 may comprise a user's exhaled breath and the apparatus 1 may be for sensing one or more analytes 9 within the user's breath. The sensing of the analytes 9 may be used for diagnostic purposes, to analyse the health of the user, or for any other suitable purpose. Water or humidity in the user's breath may be used to activate the power source 3 and provide power to the one or more sensors 5. As the power source 3 is activated by the same sample 7 that is analysed by the sensor 5 this removes the need for any additional power source to be provided. This may enable a low cost and/or disposable sensing device to be provided.

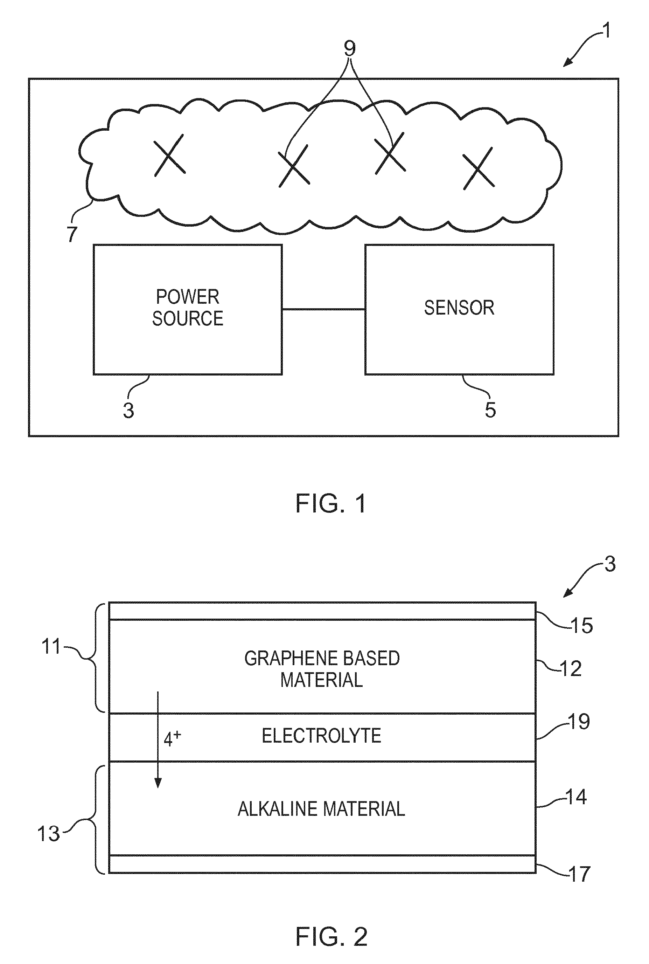

Fig. 1 schematically illustrates an apparatus 1 according to examples of the disclosure. The apparatus 1 comprises a power source 3 and at least one sensor 5. It is to be appreciated that the apparatus 1 could comprise additional components in other examples of the disclosure. One or more of such apparatus 1 may be provided within a sensing device.

The power source 3 comprises means arranged to provide power to the other components of the apparatus 1 such as the one or more sensors 5. The power source 3 may be arranged to provide electrical power to the other components of the apparatus 1. A direct electrical connection may be provided between the power source 3 and the other components of the apparatus 1. The direct electrical connection may enable charge to be transferred between the power source 3 and the other components.

In examples of the disclosure the power source 3 is arranged to be activated in response to exposure to a sample 7. The sample 7 may comprise a fluid such as a gas. The sample 7 comprises water in a gaseous phase to enable the power source 3 to be activated. In some examples the sample 7 may be a user's exhaled breath. The power source 3 is positioned within the apparatus 1 so that when the sample 11 is introduced into the apparatus 1 the power source 3 is exposed to the sample 7.

In examples of the disclosure the power source 3 comprises graphene. The graphene may be provided in a graphene based material such as graphene oxide or reduced graphene oxide. The graphene may be provided in an electrode. When the graphene is exposed to the water in the sample 7 this causes protons to be released by the graphene within the electrode and so enables power to be provided. Fig. 2 illustrates an example power source 3 in more detail.

The sensor 5 comprises any means which may be arranged to provide an electrical output in response to the presence of an analyte 9 within a sample 7. The analyte 9 could be a chemical or a group of chemicals. In examples of the disclosure the sensor 5 comprises graphene.

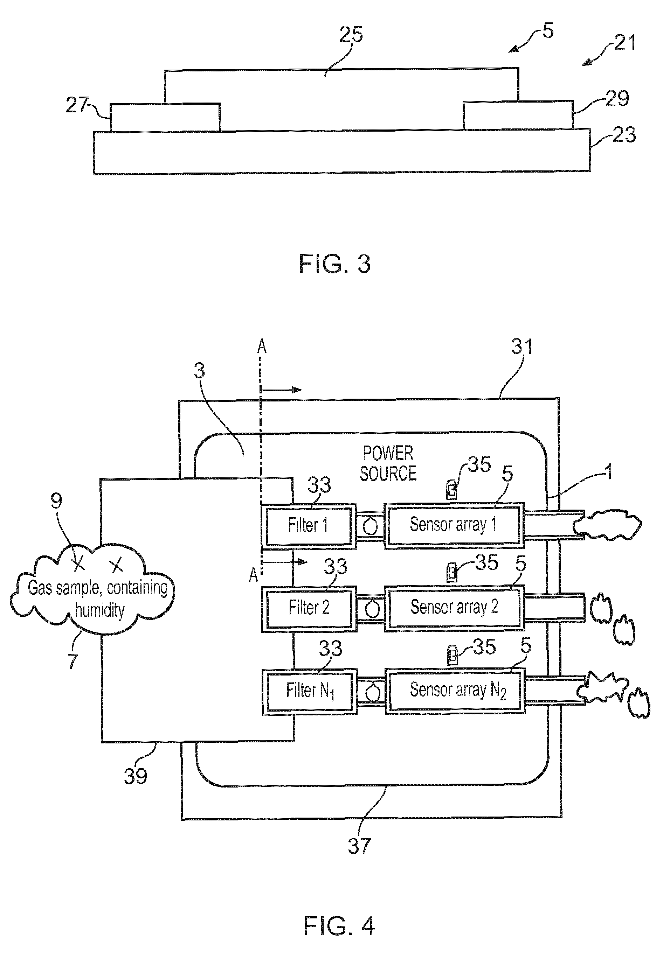

The sensors 5 are positioned within the apparatus 1 so that the sensors 5 are also exposed to the sample 7 when the sample is introduced into the apparatus 1. The sample 7 may comprise one or more analytes 9. The analytes 9 within the sample 7 may interact with the graphene within the sensor 5. This interaction may change the conductivity of the sensor 5. This produces a change in the electrical output of the sensor 5 which can be measured. Fig. 3 illustrates an example sensor 5 in more detail.

The power source 3 is arranged within the apparatus 1 to provide power to the sensor 5. A direct electrical connection may be provided between the power source 3 and the sensor 5. The direct electrical connection may provide a path for direct current between the power source 3 and the sensor 5. The power provided by the power source 3 may enable a voltage to be provided to the sensor 5. The power provided by the power source 3 may enable a change in the electrical output of the sensor 5 to be measured. The change in electrical output may be caused by the presence of an analyte 9.

In the example of Fig. 1 the apparatus 1 comprises one power source 3 and one sensor 5. It is to be appreciated that other numbers of power sources 3 and/or sensors 5 may be provided in other examples of the disclosure.

Fig. 2 schematically illustrates a power source 3 that may be provided within the apparatus 1 in some examples of the disclosure. In the example of Fig. 2 the power source 3 comprises a first electrode 11, a second electrode 13, and an electrolyte 19.

The first electrode 11 comprises graphene 12 and a first charge collector 15. In the example of Fig. 2 the graphene 12 may comprise any suitable graphene based material such as graphene oxide. The graphene oxide may be formed from an ink which is deposited onto the first charge collector 17. Other graphene based materials may be used in other examples of the disclosure.

The graphene 12 in the first electrode 11 may be arranged to have a low pH. The graphene 12 may be arranged to have an acidic pH. In the example power source 3 of Fig. 2 the first graphene 12 may be arranged to have a pH of 4. Other values of pH may be used in other examples of the disclosure

The first charge collector 15 is positioned adjacent to the graphene 12. The first charge collector 15 is positioned adjacent to the graphene 12 so that charge may be transferred from the first charge collector 15 to the graphene 12.

The first charge collector 15 may comprise a thin layer of conductive material. The first charge collector 15 may comprise gold, chromium, silver, aluminum, copper, indium tin oxide or any other suitable material or combinations of material.

The first electrode 11 may form an anode of the power source 3.

The second electrode 13 may comprise a layer of alkaline material 14 and a second charge collector 17. The layer of alkaline material 14 may be formed from any suitable alkaline material. In some examples the layer of alkaline material 14 may comprise potassium hydroxide coated onto a stainless steel mesh, graphene oxide or any other suitable material.

The layer of alkaline material 14 is arranged so that the second electrode 13 is arranged to have a pH higher than the first electrode 11. In the example power source 3 of Fig. 2 the second electrode 13 may be arranged to have a pH of 13 of 14. Other values of pH may be used in other examples of the disclosure.

The second charge collector 17 is positioned adjacent to the layer of alkaline material 14. The second charge collector 17 is positioned adjacent to the layer of alkaline material 14 so that charge may be transferred from the second charge collector 17 to the layer of alkaline material 14.

The second charge collector 17 may comprise a thin layer of conductive material. The second charge collector 17 may comprise gold, chromium, silver, aluminum, copper, indium tin oxide or any other suitable material or combinations of material.

The second electrode 13 may form a cathode of the power source 3.

In the example of Fig. 2 the power source 3 comprises an electrolyte 19. The electrolyte 19 is positioned between the first electrode 11 and the second electrode 13. The electrolyte 19 may comprise means for charge transfer from the first electrode 11 to the second electrode 13. In the example of Fig. 2 the electrolyte 19 may comprise means for enabling protons to be transferred from the first electrode 11 to the second electrode 13.

The electrolyte 19 is positioned between the first electrode 11 and the second electrode 13 so that the electrolyte 19 is in direct contact with the graphene 12 of the first electrode 11 and the layer of alkaline material 14 of the second electrode 13. The electrolyte 19 may form a junction between the first electrode 11 and the second electrode 13.

The electrolyte 19 may comprise a proton conductor film or membrane. The electrolyte 19 may comprise any suitable material such as sulfonated tetrafluoroethylene based fluoropolymer-copolymer (NAFION). In other examples the electrolyte 19 may comprise a liquid electrolyte 19 or any other suitable material.

When the first electrode 11 of the power source 3 is exposed to water the water reacts with the graphene oxide in the electrode 11 to generate protons. The protons may be generated by the reaction:

GO+H₂O→mGO⁻+H₃O+protons

where mGO⁻ is modified graphene oxide. The protons are transferred from the first electrode 11 to the second electrode 13 as the power source 3 attempts to reach equilibrium. The migration of the protons between the electrodes 11, 13 generates a voltage and so enables electrical power to be provided by the power source 3. In the example power source 3 of Fig. 2 the protons are transferred from the first electrode 11 to the second electrode 13 via the electrolyte 19. Therefore the power source 3 uses water to generate power from a graphene based material.

The water used to generate the power may come from humid air or any other suitable source. In some examples the power source 3 may be activated by exposing the power source 3 to humid air. The humid air could be the exhaled breath of a user.

The example power source 3 of Fig. 2 is a dynamic power source 3. The dynamic power source can be regenerated after discharge by continuous chemical reactions. The power source 3 may be automatically charged back to open circuit voltage without an external energy input.

It is to be appreciated that other arrangements of a power source 3 may be used in other examples of the disclosure. For instance, in some examples the power source 3 might not comprise an electrolyte 19. In such examples the first electrode 11 may be provided in direct contact with the second electrode 13.

Fig. 3 schematically illustrates a sensor 5 which may be used in some examples of the disclosure. In the example of Fig. 3 the sensor comprises a variable resistor 21. Other types of sensor 5 may be used in other examples of the disclosure.

The variable resistor 21 may be positioned within the apparatus 1 so that when the apparatus 1 is exposed to the sample 7 analytes 9 within the sample 7 may interact with the variable resistor 21. The interaction of the analytes 9 with the variable resistor 21 causes a change in the resistance of the variable resistor 21.

In the example of Fig. 3 the variable resistor 21 is mounted on a substrate 23. The variable resistor 21 comprises a graphene channel 25, a source electrode 27 and a drain electrode 29.

The substrate 23 may be any suitable substrate 23 which comprises a surface onto which the variable resistor 21 may be mounted. In some examples the variable resistor 21 may be fabricated on the surface of the substrate 23. In other examples the variable resistor 21 may be fabricated on another surface and then transferred to the substrate 23. In some examples of the disclosure the power source 3 and other components of the substrate 3 may be provided on the same substrate 23.

The substrate 23 may comprise any suitable material. In some examples the substrate 23 may comprise silicon. In some examples the substrate 23 may comprise an elastomeric material, a silicone based elastomer such as polydimethylsiloxane (PDMS), paper or any other suitable material.

The source electrode 27 and the drain electrode 29 may be formed from any suitable conductive material. In some examples the source electrode 27 and the drain electrode 29 may be formed from silver, gold, indium tin oxide or any other suitable material. In some examples the electrodes 27, 29 may be fabricated on the surface of the substrate 23. For instance the electrodes 27, 29 may be printed on the surface of the substrate 23.

The graphene channel 25 extends between the source electrode 27 and the drain electrode 29. The graphene channel 25 may be arranged in electrical contact with the source electrode 27 and the drain electrode 29 so that the graphene channel 25 provides a conductive path between the source and drain electrodes 27, 29.

The graphene within the graphene channel 25 may be provided in a thin layer. In some examples the graphene channel 25 may have a thickness in the range of nanometers. In some examples the graphene channel 25 may comprise an atomic monolayer.

The graphene channel 25 may comprise any suitable graphene based material. For example the graphene channel 25 may comprise at least one of graphene, reduced graphene oxide (rGO), functionalised graphene, carbon nanotubes, functionalised rGO, functionalised carbon nanotubes or any other suitable graphene based material. The material that is used for the graphene channel 25 and/or the material that is used to functionalise the graphene in the graphene channel 25 may depend on the analyte 9 that is to be detected by the sensor 5. For instance if the sensor 5 is to be used to detect nitrogen dioxide the graphene may be functionalise with aluminium nanoparticles or reduced graphene oxide may be functionalised with zinc oxide.

The electrical parameters of the graphene channel 25 may be affected by the exposure of the graphene channel 25 to analytes 9 within the sample 7. In some examples the analyte 9 within the sample may attach or be absorbed by the graphene based material in the graphene channel 25. The absorption of the analytes 7 affects the charge mobility within the graphene based material and so affects the electrical conductivity of the graphene channel 25. The electrical conductivity can be measured by measuring the resistance between the two electrodes 27, 29. The resistance of the variable resistor 21 therefore gives an indication of whether or not an analyte 9 is present.

It is to be appreciated that the sensor 5 of Fig. 3 is an example and other variations and types of sensors 5 may be used in other examples of the disclosure. For instance in some examples the sensor 5 could comprise a field effect transistor, a capacitive sensor or any other suitable sensor 4.

Fig. 4 illustrates a sensing device 31 for sensing the presence of analytes 9 within a sample 7. The sensing device 31 may comprise an apparatus 1 comprising a power source 3 and a plurality of sensors 5 as described above. Corresponding reference numerals are used for corresponding features. The example sensing device 31 of Fig. 4 also comprises a plurality of filters 33 and a plurality of ultraviolet light sources 35.

In the example of Fig. 4 the sensing device 1 comprise a plurality of sensors 5. Three sensors 5 are illustrated in Fig. 4 however it is to be appreciated that the sensing device 31 may comprise any number of sensors 5.

In some examples of the disclosure two or more of the sensors 5 may be arranged to detect the same analyte 9. This may enable a more reliable detection of the presence of the analyte 9 to be obtained. In some examples two or more of the sensors 5 may be arranged to detect the same analyte 9 but may be arranged to have different sensitivities. This may enable the amount of analyte 9 within the sample 7 to be quantified. For instance a first sensor 5 may have a higher sensitivity to an analyte than a second sensor 5. The first sensor 5 may be arranged to provide an output signal if the level of the analyte 9 in the sample 7 exceeds a first threshold. The second sensor 5 may be arranged to provide an output if the level of the analyte 9 in the sample 7 exceeds a second threshold. The second threshold may be higher than the first threshold and enables information about the amount of analyte 9 in the sample 7 to be obtained.

In some examples different sensors 5 within the apparatus 1 may be arranged to detect different analytes 9 within the sample 7. This may enable the presence of a plurality of different analytes 9 to be detected. The detection of the presence of different analytes 9 may enable a complex analysis of the sample 7 to be made.

In the example of Fig. 4 the sensing device 31 comprises a single power source 3. The same power source 3 may be arranged to provide power to the plurality of sensors 5 and also to other components of the sensing device 31 such as the ultraviolet light sources 35.

The sensing device 31 comprises a plurality of filters 33. A filter 33 is provided for each of the sensors 5. The filters 33 are positioned within the sensing device 31 so that the sample 7 passes through a filter 33 before it is exposed to the sensors 5.

The filters 33 may comprise any means which may be arranged to prepare the sample 7 before it is exposed to the sensors 5. The filters 33 may comprise chemical filters, mechanical filters or any other suitable means.

In some examples the filters 33 may comprise means which may be arranged to remove one or more unwanted analytes 9 from the sample 7. The filters 33 may be arranged to remove chemicals which could interact with the sensors 5 and prevent the sensors 5 from detecting the analyte 9 that the apparatus 1 is intended to detect. For instance, in examples where the sample 7 comprises a user's breath the filter 33 may be arranged to remove the water from the sample 7 as the water could react with the graphene in the sensor 5.

In some examples the filter 33 may be arranged convert a first analyte into a second analyte. For instance, where the sample 7 comprises a user's breath the apparatus 1 may be arranged to provide an indication of the presence of nitric oxide (NO). However nitric oxide may be difficult for the sensor 5 to detect and so the filter 33 may be arranged to convert the nitric oxide in the sample 7 to nitrogen dioxide (NO₂) which may be more easily absorbed by the graphene in the sensor 5 and so may be detected more easily. It is to be appreciated that the filters 33 may be arranged to convert different analytes 9 in different examples of the disclosure.

The ultraviolet light sources 35 may comprise any ultraviolet light emitting diode or any other suitable means which may be used to provide ultraviolet light.

The ultraviolet light sources 35 may be positioned within the sensing device 31 so that ultraviolet light that is emitted by the ultraviolet light source 35 is incident on the plurality of sensors 5. The ultraviolet light sources 35 may be positioned so that the ultraviolet light that is emitted by the ultraviolet light source 35 is incident on the graphene channel 25 of the sensors 5.

In the example of Fig. 4 an ultraviolet light source 35 is provided for each of the sensors 5. The sensing device 31 of Fig. 4 comprises the same number of ultraviolet light sources 35 as sensors 5. In other examples of the disclosure the number of ultraviolet light sources 35 and sensors 5 could be different. For example one ultraviolet light source 35 may be positioned to provide ultraviolet light to two or more sensors 5. This may reduce the number of ultraviolet light sources 35 needed within the sensing device 31.

The ultraviolet light source 35 may be arranged to clean the sensor 5 before the sensor 5 is exposed to the sample 7. The ultraviolet light may cause the desorption and/or removal of any analytes 9 or contaminants that are currently within the graphene channel 25. This may ensure that there are no impurities or contamination within the sensor 5 and may ensure that the sensing device 31 provides a reliable result.

In some examples the ultraviolet light source 35 may be arranged to clean the sensor 5 after the sensor 5 after the sensor 5 has been exposed to the sample 7. This may enable the apparatus 1 to be used more than once.

The ultraviolet light source 35 may be coupled to the power source so that the power source 3 provides power to the ultraviolet light source 35. This may ensure that the power source 3 is the only power source 3 needed within the sensing device 1.

In the example of Fig. 4 the sensing device 31 also comprises a chamber 37. The chamber 37 comprises any means which may be arranged to house the power source 3 and the sensors 5 and the other components of the sensing device 31.

The chamber 37 may comprise an impermeable material which may prevent unwanted chemicals and analytes from entering the chamber 37. For instance, the chamber 37 may be impermeable to analytes such as a water which would activate the power source 3 when the power source 3 is not needed.

In some examples the chamber 37 may be expandable. The walls of the chamber 37 may expand when the sample 7 is introduced in to the chamber 37. This may ensure that the size of the sensing device 31 is reduced when the sensing device 31 is not in use. This may make the storage of such sensing devices 31 easier. In other examples the chamber 37 may have a fixed size so that that size of the housing stays the same when the sample is introduced to the chamber 37.

The chamber 37 also comprises a channel 39. The channel 39 may comprise any means which enables a sample 7 to be introduced into the chamber 37. The channel 39 may be sealed prior to use to prevent unwanted chemicals from being introduced into the chamber 37.

In the example of Fig. 4 the channel 39 guides the sample 7 to the filters 33. Once the sample has passed through the filters a plurality of smaller channels guide the filtered sample to the plurality of sensors. The channel 39 may also be arranged to guide the sample 7 to the power source to enable the water in the sample 7 to activate the power source 3.

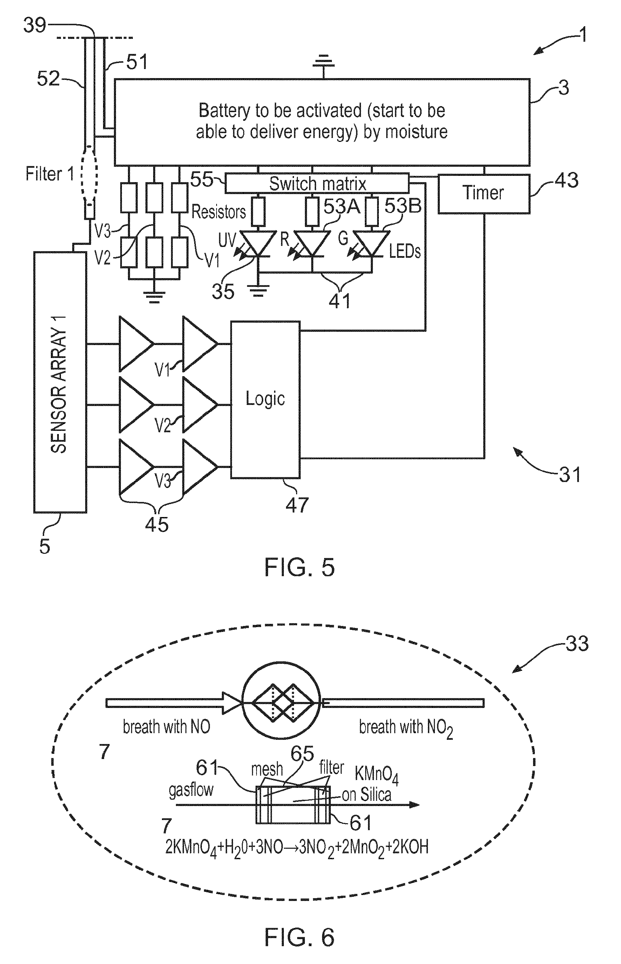

Fig. 5 illustrates a circuit diagram representing components of a sensing device 31 according to examples of the disclosure. The sensing device 31 could be a sensing device 31 as illustrated in Fig. 4. The sensing device 31 of Fig. 5 comprises a power source 3, one or more sensors 5, a filter 33, an ultra violet light source 35, and a channel 39 which may be as described above. Corresponding reference numerals are used for corresponding features. The sensing device of Fig. 5 also comprises a plurality of indicators 41, a switch matrix 55, a timer 43, amplifiers 45 and logic circuitry 47. Other components may be provided in other examples of the disclosure. For instance, the illustrated components may be housed within a chamber 37.

The line A-A may correspond to the line A-A in Fig. 4.

The channel 39 is arranged to enable a sample 7 to be provided to both the power source 3 and the sensors 5. In the example of Fig. 5 the channel 39 comprises two conduits 51, 52. The first conduit 51 guides a portion of the sample 7 to the power source 3 and a second conduit 52 guides a portion of the sample 7 to the one or more sensors. A filter 33 is provided in the second conduit 52 to enable the sample 7 to be filtered before it is provided to the sensor 5.

The sensing device 31 of Fig. 5 comprises a plurality of indicators 41. The indicators 41 may comprise any means which may enable information to be provided to a user of the sensing device 31. The indicators 41 may be arranged to provide information indicative of the status of the sensing device 31, the status of one or more components of the sensing device 31, whether or not an analyte 9 has been detected or any other suitable information. In the example of Fig. 5 the indicators 41 comprise a plurality of light emitting diodes 53. The light emitting diodes 53 are positioned in the sensing device 31 so that a user can view when they have been illuminated. Other indicators 41 may be used in other examples of the disclosure, for instance, in some examples the sensing device 31 may comprise a display, a speaker for providing an audio output or any other suitable means for providing an indication.

In the example of Fig. 5 the indicators 41 comprise two light emitting diodes 53A, 53B. Other numbers of light emitting diodes 53 may be used in other examples of the disclosure. The plurality of light emitting diodes 53 may have different colours to enable different information to be indicated to the user. In the example of Fig. 5 one of light emitting diodes 53A is red and one of the light emitting diodes 53B is green. The apparatus 1 may be arranged so that the green light emitting diode 53B is illuminated if the sensor 5 detects the presence of an analyte 9 is above a threshold and the red light emitting diode 53B is illuminated if the sensor 5 does not detect that the presence of the analyte 9 is above a threshold.

Other arrangements of the light emitting diodes 53 could be used in other examples of the disclosure. For instance, in some examples the first light emitting diode 53 could be illuminated to indicate that the power source 3 has been activated and one or more other light emitting diodes 53 could be illuminated to provide an indication of the analytes 9 detected by the sensors 5.

A switching matrix 55 is provided between the power source 3 and the indicators 41 and the ultra violet light source 35. The switching matrix 55 may comprise any means which may be arranged to control when power is provided from the power source 3 to the indicators 41 and ultra violet light source 35. The switching matrix 55 may comprise switching circuitry, controlling circuitry or any other suitable means.

The switching matrix 55 may be arranged to enable each of the indicators 41 and the ultra violet light source 35 to be controlled independently. This may enable one of the indicators 41 to be switched on without switching on the other indicators 41. This may also enable the ultraviolet light source 35 to be switched on at a different time to the indicators 41. For instance, the ultraviolet light source 35 may be arranged to be switched on before the sensor 5 is exposed to the sample 7 so that the ultra violet light may clean the sensor 5. The indicators 41 may be arranged to be switched on after the sensor 5 has been exposed to the sample 7 so that the indicators 41 can provide an indication of the results of the sensors 5.

The one or more sensors 5 are coupled to the logic circuitry 47 by one or more amplifiers 45. The amplifiers 45 may comprise any means which may be arranged to amplify the output signals of the sensors 5.

The logic circuitry 47 may comprise any means which may be arranged to detect whether an output signal of a sensor 5 is above or below a threshold. The logic circuitry 47 is arranged to provide an output signal which is dependent upon whether or not the output signal of the sensor is above or below a threshold. The output signal from the logic circuitry 47 is provided to the switch matrix 55 to control the illumination of the indicators 41.

In some examples the logic circuitry 47 may provide a plurality of output signals. The plurality of output signals may provide an indication of whether or not each of the signals provided by a plurality of sensors 5 is above or below a threshold. The plurality of output signals may then be used to control the status of a plurality of indicators 41. In other examples the logic circuitry 47 may receive a plurality of input signals from a plurality of different sensors 5. The logic circuitry 47 may be arranged to process the plurality of input signals and provide a single output signal to the switching matrix 55.

The timer 43 may comprise any means which may be used to control the timing and synchronisation of components within the sensing device 31. The timer 43 may be coupled to the power source 3 so that the timer 43 can be powered by the power source 3. The timer 43 is also coupled to the switch matrix 55 and the logic circuitry 47 to enable the timer 43 to provide timing signals to the switch matrix 55 and the logic circuitry 47.

Fig. 6 illustrates an example filter 33 that may be used in some examples of the disclosure. The example filter 33 of Fig. 6 comprises a chemical filter 65. The chemical filter 65 is arranged to convert nitric oxide in the sample 7 to nitrogen dioxide. The sample 7 may comprise a user's exhaled breath. The presence of nitric oxide may be used to provide an indication of the health of the user.

In the example of Fig. 6 the filter 33 comprises a first mesh 61 and a second mesh 63. A chemical filter comprising KMnO₄ coated on silica is positioned between the first mesh 61 and the second mesh 65, the chemical filter 65 is arranged to convert the nitric oxide to nitrogen dioxide according to the reaction:

2 KMnO₄+H₂O+3NO₂→3NO₂+2MnO₂+2KOH

Other filters 33 may be used in other examples of the disclosure.

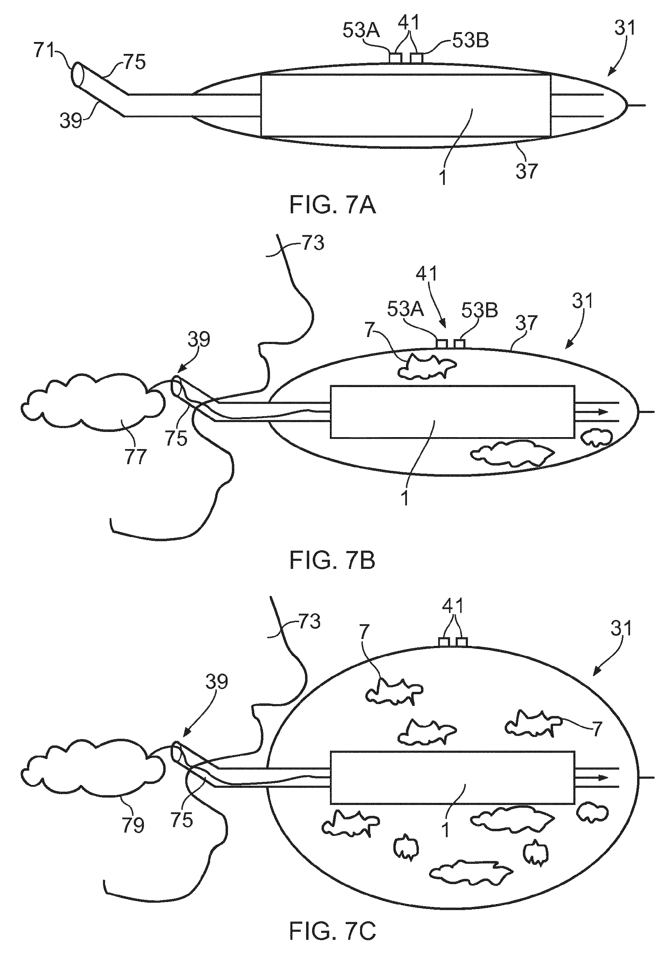

Figs. 7A to 7C illustrate an example sensing device 31 in use. The sensing device 31 may comprise an apparatus 1 which may be as described above. Components of the apparatus 1 are housed within a chamber 37 and a channel 39 is arranged to enable a sample 7 to be introduced into the chamber 37.

In the example of Figs. 7A to 7C the sensing device 31 is arranged to analyse a user's exhaled breath. For example the sensing device 31 may be arranged to analyse the amount of nitric oxide in the user's exhaled breath. The amount of nitric oxide in the user's exhaled breath may be used to test for asthma or other physical conditions which cause inflammation of the user's airway. The fraction of exhaled nitric oxide may be used for the diagnosis and/or the treatment of such conditions.

The sensing device 31 comprises a channel 39 which enables a sample 7 to be introduced to the chamber 37. In the example of Figs. 7A to 7C the sensing device 31 is arranged to analyse a user's exhaled breath and the channel 39 comprises a mouthpiece 75. The mouthpiece 75 may be a section of the channel 39 which is sized and shaped so as to fit into the mouth of a user 73.

In the example of Figs. 7A to 7C the sensing device 31 comprises a plurality of indicators 41. The indicators 41 are provided in the walls of the chamber 37 so that a user 73 can view the indicators 41 during use.

In the examples of Figs. 7A to 7C the indicators 41 comprise a red light emitting diode 53A and a green light emitting diode 53B. Other indicators 41 may be used in other examples of the disclosure.

Fig. 7A illustrates the sensing device 31 before it has been used. A lid 71 is provided over the end of the mouthpiece 75. The lid 71 may comprise any means which seals the mouthpiece 75 and prevents unwanted fluids from entering the channel 39 when the apparatus 1 is not in use.

The chamber 37 of the sensing device 31 comprises expandable walls. In the example of Fig. 7A, before the sensing sending device 31 has been used the expandable walls are in an unexpanded state. This provides a compact apparatus.

The sensing device 31 may be arranged to prevent contaminants from contacting the sensors 5 before the sensing device 31 is used. In some examples a vacuum may be provided within the chamber 37 to ensure that no contaminants are provided within the chamber. In other examples the chamber 37 may comprises dry air or any other inert gas which will not interact with the sensor 5.

Before the apparatus 1 is used the power source 3 is not activated. No power is provided to any of the components within the sensing device 31. As no power is provided by the power source 3 neither of the light emitting diodes 53 will be illuminated. If one or both of the light emitting diodes 53 is illuminated this may provide an indication that a seal of the sensing device 31 has failed and the air or other contaminants have entered the sensing device 31.

In Fig. 7B a user 73 is using the sensing device 31. In Fig. 7B the user 73 has removed the lid 71 and positioned the mouthpiece 75 into their mouth. The user 73 has exhaled a first breath 77 into the mouthpiece.

The first breath 77 provides a sample 7 which activates the power source 3. The first breath 77 also expands the walls of the chamber 37 and causes the chamber 37 to become partially inflated.

The first breath 77 may also activate the ultra violet light source 35. When the power source 3 is activated this may enable power to be provided to the ultra violet light source 35. The ultra violet source 35 may illuminate the sensor 5 with ultraviolet light. This cleans the sensor 5 in preparation for the exposure of the sensor to the sample 7. The ultraviolet light source 35 may clean the sensor 5 by accelerating the desorption of the analyte and any other chemicals from the graphene in the sensor 5.

In Fig. 7B both of the light emitting diodes 53 may be illuminated. This may provide an indication to the user 73 that the power source 3 has been activated by the first breath 77. The logic circuitry 47 and the switching matrix 55 may control the light emitting diodes 53 so that they are only illuminated once power has been provided to the one or more sensors 5. This provides an indication to the user that the sensing device 31 is ready for the user 73 to provide a second breath.

In Fig. 7C the user 73 has exhaled a second breath 79 into the mouthpiece 75. The second breath 79 provides a sample 7 which is analysed by the sensors 5 of the apparatus 1. The second breath 79 also further expands the walls of the chamber 37 and causes the chamber 37 to become fully inflated. The second breath 79 may also enable further power to be provided by the power source 3

In Fig. 7C the light emitting diodes 53 that are illuminated depends on the output signal provided by the sensor 5. For example, if the sensor 5 detects that the level of nitric oxide exceeds a threshold the green light emitting diode 53B might be illuminated. If the sensor 5 detects that the level of nitric oxide does not exceed a threshold the red light emitting diode 53A might be illuminated. This may provide a simple indication of the level of nitric oxide in the exhaled breath 79

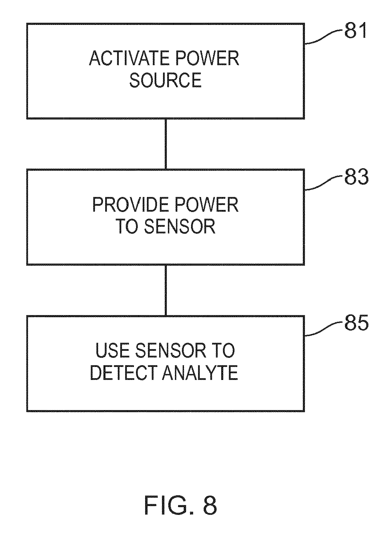

Fig. 8 illustrates a method. The method may be implemented using the apparatus 1 and sensing devices 31 described above.

The method comprises, at block 81, activating a power source 3 comprising graphene wherein the power source 3 is activated by water from a sample 7. At block 83 the method comprises providing power 3 from the power source 3 to at least one sensor 5 in response to exposure to the sample 7 and at block 85 the method comprises using the at least one sensor 5 to detect one or more analytes 9 within the sample 7.

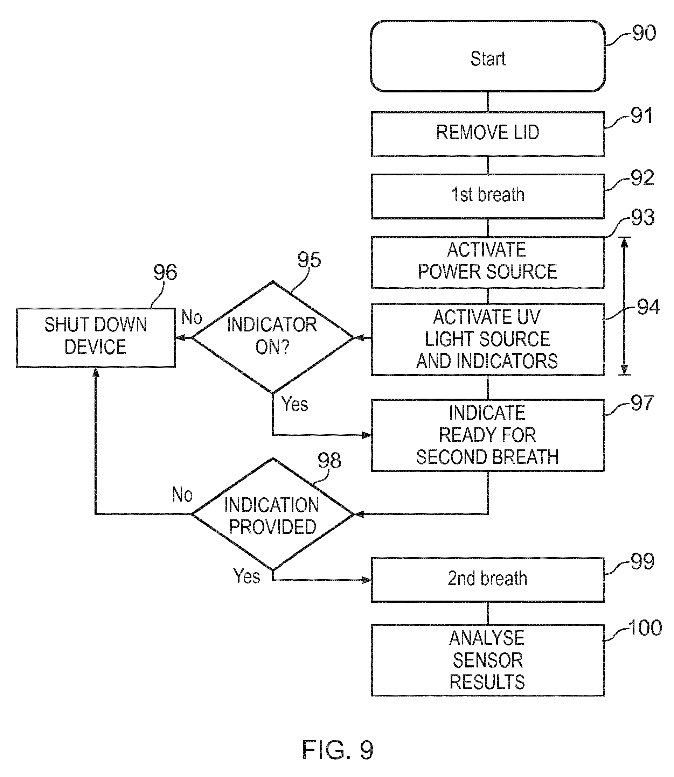

Fig. 9 illustrates another method which may be implemented using the apparatus 1 and sensing devices 31 described above. In the example of Fig. 9 the sensing device 31 is arranged to analyse a user's exhaled breath. Other sensing devices 31 may be arranged to analyse other types of sample 7.

At block 90 the user starts to use the sensing device 31. Before the user begins to use the sensing device 31 the sensing device 31 may be arranged as shown in Fig. 7A. Other arrangements of sensing devices 31 may be used in other examples of the disclosure.

At block 91 the user 73 has removed the lid 71 from the mouthpiece 75 and at block 92 the user exhales a first breath 77 into the mouthpiece 75.

The first breath 77 is guided along the channel 37 to the power source 3 so that at block 93 the power source 3 is exposed to the user's breath 77. The water in the breath 77 activates the first electrode 11 of the power source 3 and enables power 3 to be provided.

At block 94 power is provided from the power source 3 to the ultraviolet light source 35 and also to the indicators 41. The ultraviolet light source 35 exposes the sensor 5 to ultraviolet light which cleans the sensor 5 in preparation for exposure to the user's breath. The ultraviolet light source 35 may be provided internally within the chamber 37 of the sensing device 31 as the user does not need to see the ultraviolet light source 35.

The indicators 41 may comprise one or more light emitting diodes 53. The illumination of the one or more light emitting diodes 53 may provide an indication to the user 73 that the power source 3 has been activated and is generating a sufficient amount of power.

At block 95 it is determined whether or not the indicator 41 has been successfully turned on. For instance in some examples the indicator 41 comprises a plurality of light emitting diodes 53. The indicator 41 may be determined to be successfully turned on if all of the light emitting diodes 53 are illuminated. The logic circuitry 47 or any other suitable means may be arranged to determine whether or not the indicator 41 has been successfully turned on.

If at block 95 it is determined that the indicator 41 has not been successfully turned on then, at block 96 the indicator 41 is completely shut down. This provides an indication to the user 73 that the sensing device 31 is not functioning. The other components of the sensing device 31 may also be shut down.

If at block 96 it is determined that the indicator 41 has been successfully turned on then, at block 97 the indicator 41 is arranged to provide an indication to the user 73 that they can exhale the second breath 79. In examples where the indicator 41 comprises a plurality of light emitting diodes 53 the indication may comprises the blinking of the light emitting diodes 53. Other indications may be used in other examples of the disclosure.

At block 98 it is determined whether or not the indicator 41 has successfully provided the indication that the user 73 can exhale the second breath 79. For instance it may be determined whether or not the light emitting diodes 53 are blinking correctly. The logic circuitry 47 or any other suitable means may be arranged to determine whether or not the indicator 41 has successfully provided the indication that the user 73 can exhale the second breath 79.

If at block 98 it is determined that the indication has not been provided correctly then at block 96 the indicator 41 is completely shut down. This provides an indication to the user 73 that the sensing device 31 device is not functioning. The other components of the sensing device 31 may also be shut down.

If at block 98 it is determined that the indication has been provided correctly then at block 99 the user 73 exhales a second breath 79 into the mouthpiece 75.

The second breath 79 is guided along the channel 37 to the one or more sensors 5 so that at block 100 the one or more sensors 5 are exposed to the user's breath 79. Any analytes 9 within the user's breath 79 will interact with the one or more sensors 5 and change the output signals provided by the sensors 5. The logic circuitry 47 analyses the output signals from the sensors 5 and controls the indicator 41 to provide an indication of whether or not the level of an analyte 9 within the user's breath 79 exceeds a threshold.

In some examples a green light emitting diode 53B may be illuminated if the level of the analyte 9 exceeds a threshold and a red light emitting diode 53A may be illuminated is the level of the analyte 9 does not exceed the threshold. The light emitting diodes 53 may be illuminated in a stable state for a predetermined length of time. For example the light emitting diodes 53 may be illuminated constantly for two minutes. This ensures that the user 73 does not get confused between a flashing state of the light emitting diodes 53 and the indication of the outcome of the analysis by the sensors 5.

In the example of Fig. 9 the sensing device 31 uses two breaths by the user 73. The first breath 77 is used to activate the power source 3 and may be used for other purposes such as to calibrate the one or more sensors 5. The second breath 79 is analysed by the sensors 5. This may enable a smaller sensing device 31 to be provided as two smaller samples 7 are used. It may also be easier for a user 73 to provide a series of short breaths. In other examples the sensing device 31 may use a single long breath to both activate the power source 3 and also to be analysed by the sensors 5.

In other examples of the disclosure the sensing device 31 could be arranged to enable the user 73 to provide more than two breaths into the chamber. This may enable more than one measurement to be made by the sensors 5. In such examples the logic circuitry 47 may be arranged to combine the results of the sensors 5 to provide a more reliable output signal. For instance the logic circuitry 47 may be arranged to determine an average output for the sensors 5 for a plurality of measurements. In such examples the ultraviolet light source 35 may be arranged to clean the sensors 5 between measurements.

In some examples the sensing device 31 may be arranged to record the measurements obtained by the sensors 5. In such examples the sensing device 31 may comprise memory circuitry which may be coupled to the logic circuitry 47 to enable the logic circuitry 47 to read from and write to the memory circuitry. The information stored in the memory circuitry may be used to analyse the output signal from the sensor 5. For example, the information may be used to find an average result from a plurality of measurements.

In the examples described above the indicators 41 comprise light emitting diodes. It is to be appreciated that other means could be used in other examples of the disclosure, for instance, in some examples the indicators 41 could comprise an electrochromatic display. The electrochromatic display may be arranged to display a colour where the colour intensity level represents the level of analyte 9 within the sample 7. The colour of the electrochromatic display will remain constant and represent the most recent result from the sensors 5 until another measurement is made. In other examples a digital display may be provided. The digital display may be arranged to display a number or other character indicative of the level of analyte 9 within the sample 7. Other means could be used in other examples of the disclosure.

Examples of the disclosure provide an apparatus 1 which may be used in sensing devices 31. The use of graphene for both the power source 3 and the sensor 5 may enable a low cost sensing device 31 to be provided. In some examples this may enable the devices 31 to be disposable so that they can be discarded after use.

As the same sample 7 is used to activate the power source 3 and for the measurements by the sensor 5 this may enable the sensing device to be used anywhere. No external power source 3 is required which may make the apparatus more versatile. This may also enable the arrangement of the sensing device 31 to be simplified. As the power source 3 is only activated when it is exposed to the sample 7 there is no requirement for an on/off switch. This may be useful in low cost and/or disposable sensing devices 31.

In some examples the apparatus 1 may comprise ultraviolet light sources 35 which are arranged to clean the sensors 5. This may improve the sensitivity and/or the response time of the sensors 5. This may provide for more accurate and/or faster analysis of the sample 7. As the ultraviolet light sources 35 are also powered by the power source 3 this does not require any additional power sources.

The sensing device 31 may be arranged to detect any suitable analyte 9 in any suitable sample 7 by appropriate selection of the materials used within the sensor 5.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a subclass of the class that includes some but not all of the instances in the class. It is therefore implicitly disclosed that a features described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
a power source comprising graphene wherein the power source is arranged to be activated by water from a sample;
at least one sensor comprising graphene arranged to detect one or more analytes within the sample; and
wherein the power source is arranged to provide power to the sensor in response to exposure to the sample.

2. An apparatus as claimed in any preceding claim wherein the sample comprises a user's breath.

3. An apparatus as claimed in any preceding claim comprising at least one filter arranged to remove analytes from the sample before the sensor is exposed to the sample.

4. An apparatus as claimed in any preceding claim comprising at least one filter arranged to convert a first analyte into a second analyte before the sensor is exposed to the sample.

5. An apparatus as claimed in any preceding claim comprising an ultraviolet light source arranged to expose the at least one sensor to ultraviolet light.

6. An apparatus as claimed in claim 5 wherein the power source is arranged to provide power to the ultraviolet light source.

7. An apparatus as claimed in any preceding claim comprising a plurality of sensors wherein the plurality of sensors are arranged to have different sensitivities and/or detect different analytes.

8. An apparatus as claimed in any preceding claim comprising one or more indicators arranged to show an output of the sensor.

9. An apparatus as claimed in claim 8 wherein the power source is arranged to provide power to the one or more indicators.

10. An apparatus as claimed in any preceding claim comprising a chamber arranged to house the power source and the one or more sensors wherein the chamber comprises a channel arranged to enable the sample to be introduced in to the chamber.

11. An apparatus as claimed in any preceding claim wherein the power source comprises a first electrode comprising graphene oxide which is arranged to generate protons in response to water from the sample.

12. An apparatus as claimed in any preceding claim wherein the at least one sensor comprises a graphene channel comprising at least one of; graphene, reduced graphene oxide, functionalized graphene, carbon nanotubes.

13. A sensing device comprising an apparatus as claimed in any preceding claim.

14. A sensing device as claimed in claim 13 wherein the sensing device is disposable.

15. A method comprising:
activating a power source comprising graphene wherein the power source is activated by water from a sample;
providing power from the power source to at least one sensor comprising graphene in response to exposure to the sample; and
using the at least one sensor to detect one or more analytes within the sample.
